# EUROPÄISCHE PATENTSCHRIFT

(11) **EP 2 077 774 B1**
(45) Veröffentlichungstag und Bekanntmachung des Hinweises auf die Patenterteilung: **10.09.2014**
(21) Anmeldenummer: 07818472.8
(22) Anmeldetag: 26.09.2007
(51) Int. Cl.: A61B 17/29, A61B 17/295, A61B 18/14, A61B 17/00, A61B 19/00

(54) **ROHRSCHAFTINSTRUMENT**
TUBULAR SHAFT INSTRUMENT
INSTRUMENT À TIGE TUBULAIRE

(30) Priorität: 05.10.2006 DE 102006047204; 05.10.2006 DE 102006047215; 05.10.2006 DE 102006046919; 05.10.2006 DE 102006046920; 29.11.2006 DE 102006056405; 14.12.2006 DE 102006059175
(43) Veröffentlichungstag der Anmeldung: 15.07.2009
(73) Patentinhaber: ERBE Elektromedizin GmbH, 72072 Tübingen (DE)
(72) Erfinder: HAFNER, Dieter, 72072 Tübingen (DE)
(74) Vertreter: Bohnenberger, Johannes
(86) Internationale Anmeldenummer: PCT/EP2007/008389
(87) Internationale Veröffentlichungsnummer: WO 2008/040486

(56) Entgegenhaltungen:
- DE-A1- 4 204 051
- DE-A1- 4 444 166
- DE-A1- 10 031 773
- US-A- 5 293 863
- US-A- 5 797 938

## Beschreibung

Die Erfindung betrifft ein Rohrschaftinstrument gemäß dem Oberbegriff des Anspruchs 1.

In der modernen Medizin versucht man im Allgemeinen, die Schädigung von intaktem Gewebe so gering wie möglich zu halten. Die minimalinvasive Chirurgie ist daher, wenn es die Umstände erlauben, meist die bevorzugte Art, mit der ein operativer Eingriff durchgeführt wird. Kleine Schnitte und eine geringe Verletzung des Gewebes führen zu einem geringeren Schmerzempfinden nach der Operation und zu einer raschen Erholung und Mobilisation des Patienten. Dies trifft auch für die laparoskopische Chirurgie zu, bei der komplexe Operationen im Bauchraum durchgeführt werden.

Für die Hersteller von medizinischen Instrumenten, stellen diese Art von Operationen und die dafür benötigten Geräte eine besondere Herausforderung dar, da ein Großteil der operativen Schritte in sehr beschränkten Räumen und ohne direkten Sichtkontakt durchgeführt werden. So müssen sich die verwendeten medizinischen Geräte nicht nur in kleinsten Räumen handhaben lassen, sondern auch so sicher funktionieren, dass eine optische Kontrolle überflüssig ist. Vorzugsweise sind die Instrumente derart beschaffen, dass dem operierenden Arzt auch ohne Sichtkontakt stets eine Rückmeldung zur Verfügung steht, die einen Rückschluss auf den Verlauf der Operation zulässt.

Dies gilt besonders für sämtliche Instrumente, die sich zum Durchtrennen von Gewebe eignen. Da für die minimalinvasive Chirurgie Skalpelle mit einer offenen Klinge eher ungeeignet sind (vgl. hierzu die DE 44 44 166 A1), greift man häufig auf scherenartige bzw. zangenartige Instrumente zurück, die zum einen beim Einführen des Instruments die Klinge abdecken und zum anderen gleichzeitig eine Haltefunktion für das zu schneidende Gewebe übernehmen.

Es hat sich als vorteilhaft erwiesen, auch bei der minimalinvasiven Chirurgie vor dem Durchtrennen eine Koagulation des Gewebes durchzuführen, um ein Einbluten zu vermeiden. Aus dem Stand der Technik ist es bekannt, Instrumente bereit zu stellen, die über integrierte Koagulations- und Schneidvorrichtungen verfügen. Hier wird das Gewebe auf einer Fixierebene in einem ersten Schritt festgeklemmt und koaguliert. In einem zweiten Schritt wird ein Messer mit einer Schneide, die im Wesentlichen senkrecht zu der Fixierebene steht und beidseitig über das Gewebe herausragt, durch das Gewebe geführt. Diese Bewegung erfolgt im Wesentlichen parallel zur Fixierebene. Dieser Stand der Technik geht beispielsweise aus der US 6 679 882 B1, EP 717 960 B1, WO-2005/004735 A1 hervor.

Aus der US 6 626 901 B1 ist es andererseits bekannt, an Stelle des Messers eine Pizza-Rollen-ähnliche Schneide über das Gewebe zu rollen und somit den Schnitt durchzuführen. Während das letztgenannte Verfahren sehr aufwändig ist und besondere Anforderungen an die Gestaltung des Instruments stellt, weisen die anderen Verfahren zahlreiche Nachteile auf. So wird durch die Bewegung des Messers das eingeklemmte Gewebe im Wesentlichen aus dem Maulteil heraus geschoben und nur ein Teil des gefassten Gewebes durchtrennt. Beim Durchtrennen, kommt es zu einer hohen punktuellen Belastung des Gewebes, wodurch wiederum bei abgenutzter Klinge kein sauberer Schnitt gewährleistet ist. Das Gewebe wird so lange gequetscht, bis es quasi zerreißt. Es besteht die Gefahr, dass sich die Schnittnaht so weit ausbreitet, dass sie über das koagulierte Gebiet reicht und der bereits durchgeführte Gefäßverschluss wieder aufreißt. Da die Klinge bzw. Schneide nur an einem Punkt an dem Gewebe ansetzt, kommt es zu einem schnellen Verschleiß der Schneide.

Aus der DE 42 04 051 A1 ist ein Rohrschaftinstrument zum Durchtrennen von Gewebe bekannt. Dieses Instrument weist einen fest mit dem Rohrschaft verbundenen Amboss mit einem spitz nach oben verlaufenden Ende auf. Dieser Amboss dient dazu, Gewebe aufzunehmen. Drehbeweglich gegenüber dem Amboss ist eine Klinge angeordnet, die sich durch eine Betätigungsstange auf den Amboss herabsenken lässt. Soweit gefasstes Gewebe nicht unmittelbar beim Herabsenken der Klinge durchtrennt werden kann, spannt sich mit zunehmender Betätigung der Betätigungsstange eine Feder, die die Klinge gegen das Gewebe vorspannt. Durch das Spannen der Feder lässt sich die Klinge in proximale Richtung zurückziehen. Der in der DE 42 04 051 A1 bereitgestellte Mechanismus ist relativ komplex und kann ein sicheres Durchtrennen des Gewebes nicht gewährleisten.

Ausgehend von der DE 42 04 051 A1 diesem Stand der Technik ist es Aufgabe der vorliegenden Erfindung, ein einfach bedienbares Rohrschaftinstrument zum Durchtrennen von Gewebe bereit zu stellen, das ein sicheres Durchtrennen mit minimaler Beschädigung des umliegenden Gewebes gewährleistet.

Diese Aufgabe wird durch ein Rohrschaftinstrument gemäß dem vorliegenden Anspruch 1 gelöst.

Die Aufgabe wird bei einem Rohrschaftinstrument, insbesondere elektrochirurgisches Rohrschaftinstrument, zum Durchtrennen von Gewebe, umfassend:
einen Rohrschaft,
ein erstes und ein zweites Maulteil mit Klemmflächen zum Fixieren von Gewebe in einer Fixierebene, wobei mindestens eines der Maulteile mit dem Rohrschaft verbunden ist,
eine Schneidvorrichtung mit einer Schneide, die zum Schneiden des fixierten Gewebes mittels einer Betätigungseinrichtung in einer Schnittrichtung bewegbar ist,
dadurch gelöst,
dass die Schneide im Wesentlichen parallel zur Fixierebene beweglich geführt und durch eine Vorspanneinrichtung beim Schneiden gegen die Fixierebene vorgespannt ist.

Ein wesentlicher Gedanke der Erfindung besteht also darin, die Schneide nicht durch die Fixierebene hindurch und somit durch das Gewebe hindurch zu bewegen, sondern diese mittels einer Führung oberhalb oder unterhalb der Fixierebene zu leiten, wobei die Schneide mittels der Vorspanneinrichtung gegen die Fixierebene gedrückt wird. Die Schneide gleitet also mit einem definierten Anpressdruck vorzugsweise in Hin- und Herbewegungen so lange über das Gewebe und somit über die Fixierebene, bis das Gewebe vollständig durchtrennt ist. Somit wird ein Durchschneiden des Gewebes und nicht ein Zerreißen durch mechanischen Druck sichergestellt. Die Kraft der Vorspanneinrichtung wirkt im Wesentlichen senkrecht zur Fixierebene.

Weiterhin umfasst die Schneide mindestens einen Abschnitt, der im Wesentlichen parallel zur Fixierebene verläuft. Denkbar ist auch eine Schneidvorrichtung mit mehreren Schneiden, wobei mehrere Abschnitte der Schneiden parallel zur Fixierebene verlaufen.

Die Vorspanneinrichtung umfasst einen elastischen Führungsdraht mit einer Biegung, der mit der Schneide im Wesentlichen starr verbunden ist und derart im Rohrschaft geführt ist, dass die Schneide gegenüber dem Rohrschaft zur Fixierebene hin vorgespannt ist. Die besagte, im Wesentlichen senkrecht zur Fixierebene wirkende Kraft kann also durch ein elastisches Betätigungselement in Form eines Führungsdrahts aufgebaut werden. Die aufgebrachte Kraft wirkt gegenüber dem Rohrschaft, in welchem der Draht geführt ist. Im einfachsten Fall weist somit der Führungsdraht einen Knick auf.

Der elastische Führungsdraht nutzt einen Abschnitt des Rohrschafts zumindest für die Kraftumlenkung bzw. Abstützung.

Vorzugsweise umfasst die Vorspannvorrichtung eine Sicke, die derart im Führungsdraht angeordnet ist, dass sie bei vorgeschobener Klinge nahe dem distalen Ende des Rohrschafts ist. Somit kann die Sicke die durch das Anpressen der Schneide auf die Schneidebene entstehende Kraft an den Rohrschaft weiter geben. Für den Benutzer des Rohrschaftinstruments dient der Führungsdraht allein zur Bewegung der Schneide in der Schneidrichtung. Im Optimalfall wirken keine Rotationskräfte auf den Benutzer, da der Rohrschaft den Anpressdruck aufnimmt. Für eine funktionale Anordnung des Führungsdrahtes ist es notwendig, dass die Sicke, sowie die Biegung im Wesentlichen in der Schneidebene liegen, die auf der Fixierebene senkrecht steht. Natürlich ist der Führungsdraht auch durch eine Führungsschiene ersetzbar, die besagte elastische Eigenschaften senkrecht zur Fixierebene aufweist. Auch in diesem Fall kann die Vorspannung durch die Form der Schiene erzeugt werden.

Vorzugsweise umfasst mindestens eines der beiden Klemmteile eine sich entlang der Schnittrichtung erstreckende Öffnung, die eine Klingenführung bildet. Die im Wesentlichen länglichen Klemmteile können paarweise ausgebildete Seitenteile umfassen, die einen Kanal aufweisen, in dem die Schneidvorrichtung entlang der Schnittrichtung geführt wird.

Vorzugsweise ist die Schneide mindestens abschnittsweise konvex gekrümmt. Die Schneide ist also derart ausgebildet, dass bei der Bewegung der Schneide in Schnittrichtung diese auf das in der Fixierebene vorgespannte Gewebe gleitet. Dies ist besonders vorteilhaft, wenn das zu durchtrennende Gewebe derart widerstandsfähig ist, dass es nicht durch einen Schnitt durchtrennt werden kann. Das Profil der Schneide wirkt in diesem Fall wie eine Rampe, die die Schneide in eine Position oberhalb der Fixierebene führt. Dort gleitet die Schneide so lange über das Gewebe, bis dies vollständig durchtrennt ist. Die Vorspanneinrichtung stellt die nötige Anpresskraft bereit.

Vorzugsweise umfasst das Rohrschaftinstrument eine insbesondere rampenförmige Klingenführung, die derart ausgebildet und angeordnet ist, dass sie die Schneide aus einer Fixierebene beabstandeten Ausgangsposition durch ein Bewegen in der Schnittrichtung auf die Fixierebene zuführt. Vorzugsweise wird also die Schneide in einer Ausgangsposition gelagert, die von der Fixierebene sowie von den Klemmflächen der Maulteile beabstandet ist. Erst nachdem das Gewebe zwischen den Klemmflächen fixiert ist, wird die Schneide durch einen Verschub parallel zur Fixierebene auf diese zugeführt. In der Ausgangsposition ist die Schneide abgeschirmt, das Gewebe kann unverletzt aufgenommen werden. Insbesondere wenn es sich bei dem Rohrschaftinstrument um ein elektrochirurgisches Instrument mit Elektroden zur Koagulation des gefassten Gewebes handelt, kann ein verfrühtes Anschneiden des Gewebes verhindert werden.

Vorzugsweise ist die Klingenführung derart ausgebildet, dass die Schneide in eine Ausgangsposition nahe der Drehachse der Maulteile, insbesondere im Rohrschaft, bringbar ist.

Vorzugsweise weisen die Klemmflächen Elektroden zum Zuführen von HF-Koagulationsströmen zu dem gefassten Gewebe auf. Somit kann das gefasste Gewebe vor oder während des Schneidens durch einen hochfrequenten Koagulationsstrom verödet werden, wodurch ein sicherer Gefäßverschluss vor oder während des Schnitts gewährleistet wird.

Vorzugsweise weist die Schneide mindestens abschnittsweise eine Mikroverzahnung auf.

Weitere vorteilhafte Ausführungsformen ergeben sich aus den weiteren Unteransprüchen.

Nachfolgend wird die Erfindung anhand von Ausführungsbeispielen beschrieben, die mittels Abbildungen näher erläutert werden. Hierbei zeigen:
- Fig. 1 ein Rohrschaftinstrument zum Durchtrennen von Gewebe;
- Fig. 2 den Werkzeugkopf des Rohrschaftinstruments aus Fig. 1, umfassend ein erstes und ein zweites Maulteil;
- Fig. 3 das zweite Maulteil in einer perspektivischen Seitenansicht;
- Fig. 4 das zweite Maulteil in einer Draufsicht;
- Fig. 5 das zweite Maulteil in einer Seitenansicht;
- Fig. 6 das erste Maulteil in einer perspektivischen Seitenansicht;
- Fig. 7 das erste Maulteil in einer Draufsicht;
- Fig. 8 das erste Maulteil in einer Seitenansicht;
- Fig. 9 eine schematische Darstellung zweier unterschiedlicher Drehgelenke;
- Fig. 10 ein Querschnitt durch den Werkzeugkopf aus Fig. 2 mit einer Schneidvorrichtung;
- Fig. 11 eine schematische Darstellung der Schneidvorrichtung;
- Fig. 12 eine schematische Darstellung der Schneidvorrichtung in einem Rohrschaft eines Rohrschaftinstruments;
- Fig. 13-15 drei Ausführungsformen einer Schneidklinge;
- Fig. 16 ein Blockdiagramm einer Schnittkontrollvorrichtung;
- Fig. 17 eine perspektivische Ansicht eines Werkzeugkopfs in einer geöffneten Stellung;
- Fig. 18 den Werkzeugkopf aus Fig. 17 in einer geschlossenen Stellung;
- Fig. 19 das zweite Maulteil mit Zugband; und
- Fig. 20 eine schematische Seitenansicht des Rohrschaftinstruments.

In der nachfolgenden Beschreibung werden für gleiche und gleich wirkende Teile dieselben Bezugsziffern verwendet.

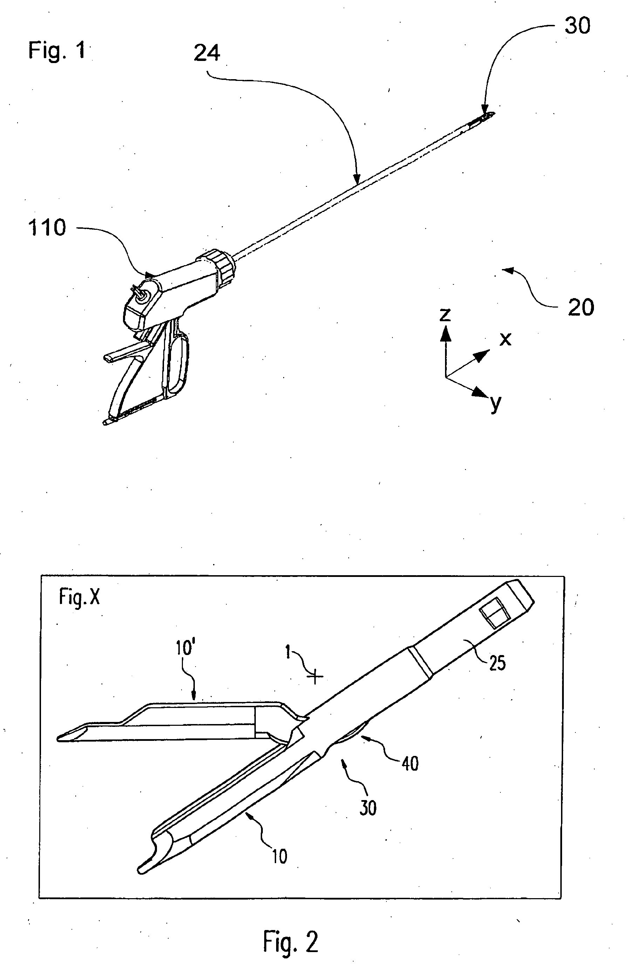

Die Fig. 1 gibt einen groben Überblick über eine Ausführungsform eines erfindungsgemäßen Rohrschaftinstruments. Sie zeigt drei funktionale Komponenten des Rohrschaftinstruments, einen Handgriff 110, einen länglichen Rohrschaft 24 und einen am distalen Ende des Rohrschafts 24 angeordneten Werkzeugkopf 30. Der Werkzeugkopf 30 stellt die eigentliche Funktionalität des Rohrschaftinstruments bereit. Er dient zum Schneiden und/oder Koagulieren von Gewebe. Der Handgriff 110 steuert die Bewegung des Werkzeugkopfs 30. Insbesondere können mittels des Handgriffs 110 Maulteile 10, 10' (vgl. Fig. 2) zum Fixieren, Koagulieren und Schneiden von Gewebe geschlossen sowie geöffnet werden.

Die Fig. 2 zeigt eine Ausführungsform eines erfindungsgemäßen Werkzeugkopfs 30, der ein erstes Maulteil 10 und ein zweites Maulteil 10' umfasst. Das erste Maulteil 10 ist ein länglicher Körper, der an seiner dem Rohrschaft 24 zugewandten Seite einen Adapter 25 aufweist, der starr mit dem Rohrschaft 24 verbunden ist. Über ein Gelenk 40 ist das zweite Maulteil 10' an dem ersten Maulteil 10 befestigt und kann aus einer geöffneten Stellung zum Ergreifen des Gewebes in eine geschlossene Stellung zum Fixieren des Gewebes gebracht werden. Das Gelenk 40 ist derart ausgebildet, dass sich eine virtuelle Drehgelenksachse 1 bzw. Drehachse außerhalb des ersten und zweiten Maulteils 10, 10' befindet. Anders als bei herkömmlichen Gelenken 40 für derartige Instrumente liegt also die Drehgelenksachse 1 nicht in dem Bereich, in dem die Maulteile 10, 10' ineinander eingreifen, oder im Rohrschaft 24, nahe der Längsachse des Rohrschafts 24. Die Mechanik des dargestellten Gelenks 40 wirkt so, dass sich eine virtuelle Drehgelenksachse 1 oberhalb der dem zweiten Maulteil 10' zugewandten Seite des Rohrschaftinstruments ausbildet.

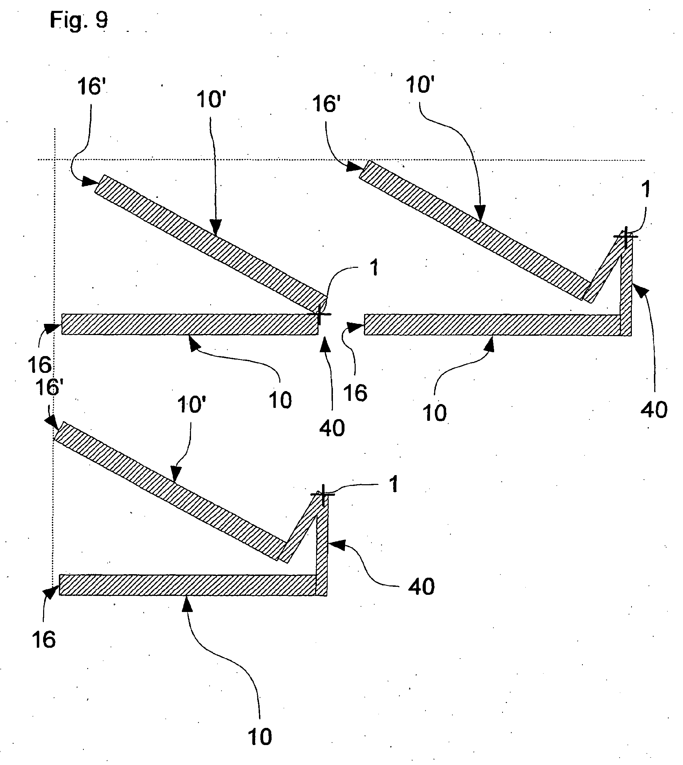

Die besonderen Vorteile einer solchen ausgelagerten Drehgelenksachse 1 zeigen sich anhand der schematischen Darstellungen der Fig. 9. In der oberen linken Ecke ist dort ein herkömmliches Drehgelenk abgebildet, dessen Drehgelenksachse 1 im Wesentlichen auf den Längsachsen der Maulteile 10 und 10' liegt. Im geöffneten Zustand ist die Spitze 16' des zweiten Maulteils 10' gegenüber der Spitze 16 des ersten Maulteils 10 zurückversetzt. Anders verhält es sich jedoch bei dem erfindungsgemäßen Gelenk 40, das schematisch in den anderen beiden Abbildungen der Fig. 9 gezeigt wird. Hier befindet sich die Drehgelenksachse 1 deutlich oberhalb der Längsachsen der beiden länglichen Maulteile 10, 10'. Bei gleicher Öffnung hinsichtlich des Winkels, den das erste Maulteil 10 zum zweiten Maulteil 10' ausbildet, liegt die Spitze 16' des zweiten Maulteils 10' auch im geöffneten Zustand im Wesentlichen auf bzw. vor einer Lotgeraden durch die Spitze 16 des ersten Maulteils 10. Öffnet man das zweite Maulteil 10' gegenüber dem ersten Maulteil 10, kommt es also nicht nur zu einer Drehbewegung, bei der sich die relative Ausrichtung des zweiten Maulteils 10' zum ersten Maulteil 10 verändert, sondern auch zu einer Längsverschiebung des zweiten Maulteils 10', die in distaler Richtung, also parallel zur Längsachse des ersten Maulteils 10 in Richtung dessen Spitze 16 orientiert ist. Umgekehrt kommt es bei einer Schließbewegung der Maulteile 10, 10' zu einer Längsverschiebung des zweiten Maulteils 10' in proximaler Richtung. Hierdurch wird Gewebe, das sich zwischen den beiden Maulteilen 10, 10' befindet, im Endeffekt in den Werkzeugkopf 30 hineingezogen. Des Weiteren ist der Hub der zweiten Spitze 16, also die Distanz zwischen der ersten und der zweiten Spitze 16, 16' bei gleichem Öffnungswinkel wesentlich größer (vgl. Fig. 9, rechts). In einem Ausführungsbeispiel verhält sich die Länge der Maulteile 10, 10' zur Distanz der Längsachse des ersten Maulteils 10 zur Drehgelenksachse ca. im Verhältnis 10:1.

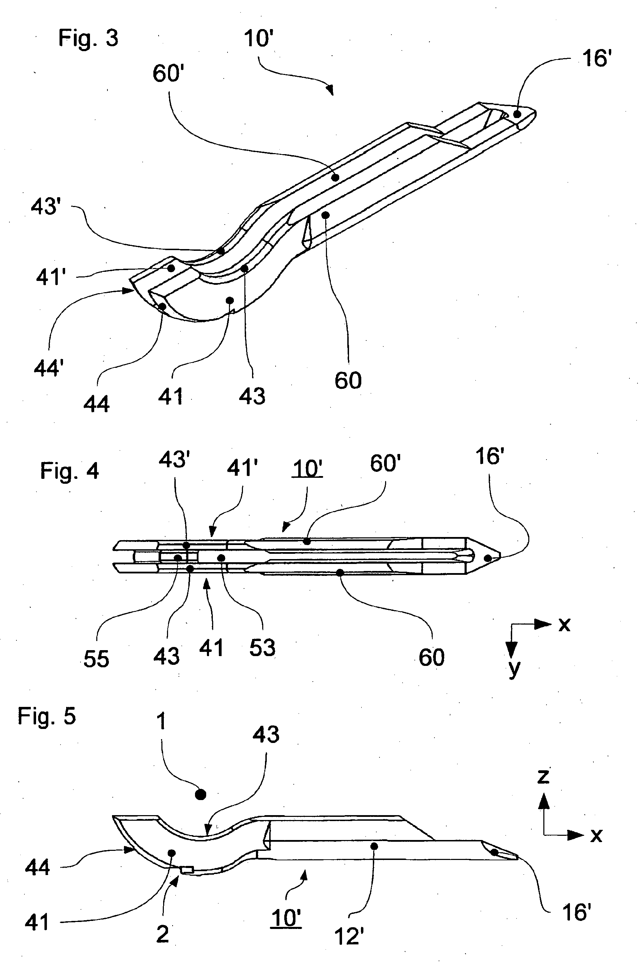

Während in der Fig. 9 die ausgelagerte Drehgelenksachse 1 zur Verdeutlichung über senkrecht an die proximalen Enden der Maulteile 10, 10' ansetzende Verlängerungen erzielt wird, ist in einer bevorzugten Ausführungsform die Drehgelenksachse 1 rein virtuell ausgebildet. Diese virtuelle Ausbildung wird durch eine Kulissenführung, wie sie im Weiteren anhand der Fig. 3-8 erläutert wird, erzielt. So weist, wie in Fig. 3 dargestellt, das zweite Maulteil 10' an seinem proximalen Ende gegenüber der Spitze 16' zwei gebogene Gelenkführungsschienen 41, 41' auf. Aus einer Draufsicht betrachtet (vgl. Fig. 4) verlaufen diese Gelenkführungsschienen 41, 41' im Wesentlichen parallel entlang der Längsachse des zweiten Maulteils 10' und sind voneinander zur Bildung eines Kanals beabstandet.

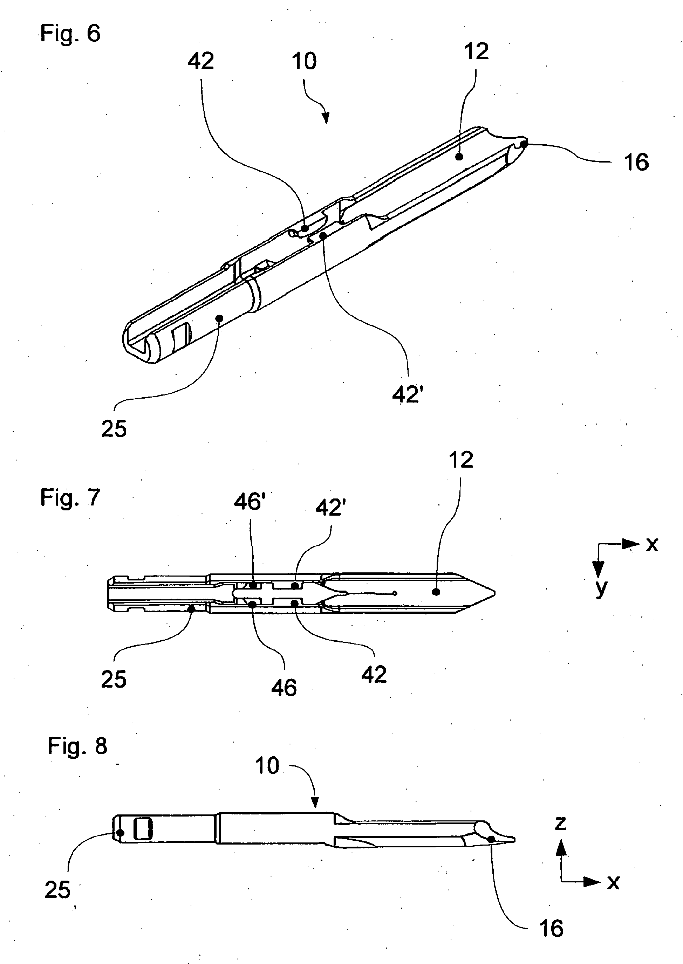

Von der Seite betrachtet (vgl. Fig. 5) weist das zweite Maulteil 10' ein löffelförmiges Profil auf. Das proximale Ende des zweiten Maulteils 10', insbesondere die Gelenkführungsschienen 41, 41', weisen also an ihrer Oberseite jeweils einen konkaven Abschnitt 43, 43' auf, der mit dem ersten Maulteil 10 in Eingriff steht. Wie anhand der Fig. 6 ersichtlich, weist hierfür das erste Maulteil 10 zwei Gelenkführungszapfen 42, 42' auf, die jeweils über einen konvexen Strukturabschnitt verfügen. Bei der Öffnungs- und Schließbewegung der Maulteile 10, 10' gleitet also der konkave Abschnitt 43 der ersten Gelenkführungsschiene 41 über den benachbarten, konvexen Abschnitt des ersten Gelenkführungszapfens 42 und der konkave Abschnitt 43' der zweiten Gelenkführungsschiene 41' über den benachbarten, konvexen Abschnitt des zweiten Gelenkführungszapfens 42'. Die Krümmung der konkaven Abschnitte 43, 43' der beiden Gelenkführungsschienen 41, 41' sowie der korrespondierenden Abschnitte der Gelenkführungszapfen 42, 42' sind maßgeblich für die Position der virtuellen Drehgelenksachse 1. Bei einer stärkeren Krümmung ist die Drehgelenkachse 1 näher beim Werkzeugkopf 30 gelegen als bei einer schwachen Krümmung. Entsprechend stark und schwach treten die hinsichtlich der Fig. 9 beschriebenen Effekte auf.

Das Führungsgetriebe bzw. Gelenk 40 hat gegenüber Gelenken, die lediglich eine punktuelle Verbindung aufweisen, des Weiteren den Vorteil einer hohen Stabilität. Durch die konvexen und konkaven Abschnitte, die in einander eingreifen, wird ein großflächiger Kontaktbereich ausgebildet und das Gelenk 40 kann wesentlich mehr Kraft aufnehmen, als ein Gelenk mit einer punktuellen Verbindung. Zur weiteren Stabilisierung des Gelenks 40 umfasst das erste Maulteil 10 ein erstes Gelenkführungslager 46 und ein zweites Gelenkführungslager 46'. Wie die Gelenkführungszapfen 42, 42' sind die Gelenklager 46, 46' wechselseitig an der Innenseite der Seitenwände des ersten Maulteils 10 angebracht.

Das erste Gelenkführungslager 46 und der erste Führungszapfen 42 sind derart voneinander beabstandet, dass sie in ihrem Zwischenraum die erste Gelenkführungsschiene 41 aufnehmen. Das erste Gelenkführungslager 46 weist einen konkaven Schnitt auf, der mit einem konvexen Abschnitt 44 der ersten Gelenkführungsschiene 41 in Eingriff steht. Beim Öffnen und Schließen des Werkzeugkopfs 30 rotiert die erste Gelenkführungsschiene 41, geführt von dem ersten Führungszapfen 42 und dem ersten Gelenkführungslager 46 um die Drehgelenksachse 1.

Ebenso rotiert die zweite Gelenkführungsschiene 41', geführt von dem zweiten Führungszapfen 42' und dem zweiten Gelenkführungslager 46' um die Drehgelenksachse 1. Hierfür sind die zweite Gelenkführungsschiene 41', der zweite Gelenkführungszapfen 42', ein konvexer Abschnitt 44' der zweiten Gelenkführungsschiene 41' und das zweite Gelenkführungslager 46' symmetrisch zu der ersten Gelenkführungsschiene 41, dem ersten Gelenkführungszapfen 42, dem konvexen Abschnitt 44 der ersten Gelenkführungsschiene 41 und dem ersten Gelenkführungslager 46 ausgebildet und angeordnet.

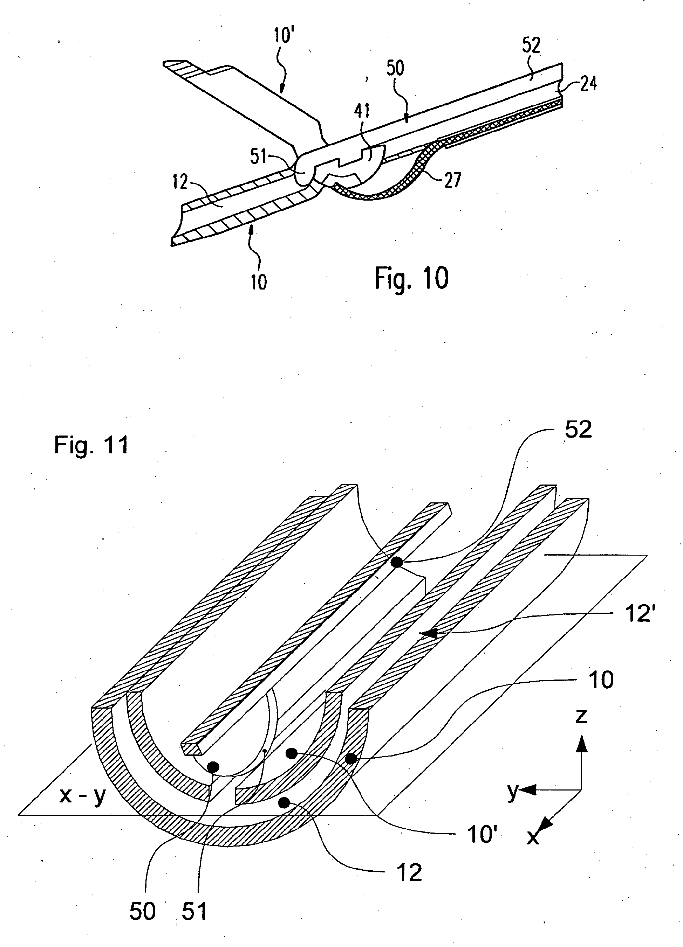

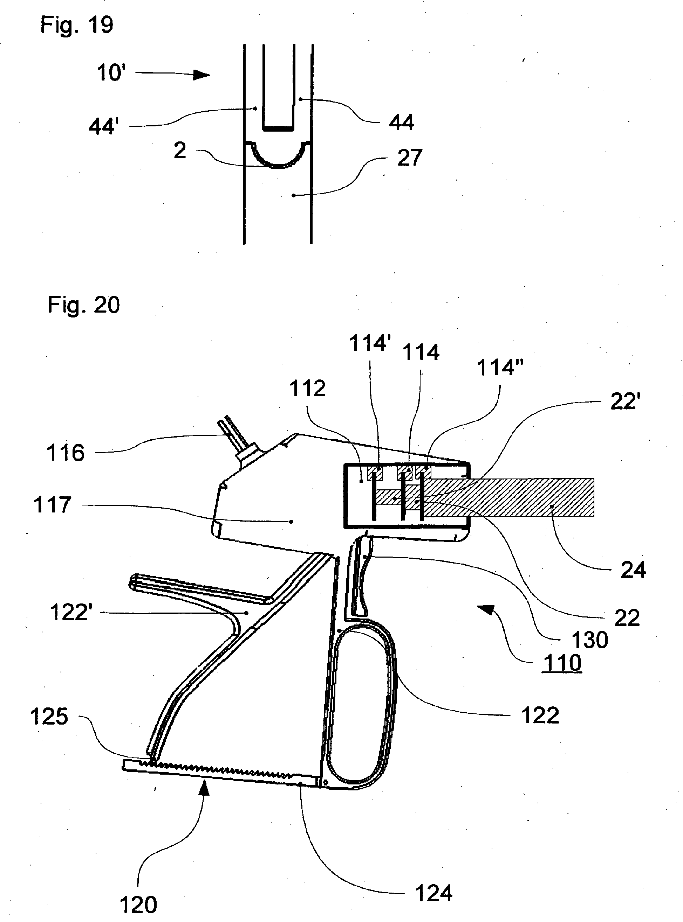

Wie in der Fig. 10 gezeigt, setzt ein Zugband 27 am proximalen Ende des zweiten Maulteils 10' an. Genauer gesagt, ist es ungefähr mittig an den konvexen Abschnitten 44, 44' der Gelenkführungsschienen 41, 41' angebracht. Hierfür weisen die Gelenkführungsschienen 41, 41' ein Profil zum Bilden einer Stoßkante 2 (Fig. 5) auf. Vorzugsweise verläuft diese Stoßkante 2 nicht geradlinig parallel zur Drehgelenkachse 1, sondern ist halbkreisförmig (vgl. Fig. 19) ausgebildet. Durch diese verlängerte Stoßkante 2, entlang der das zweite Maulteil 10' und das Zugband 27 verschweißt sind, sind die Krafteinleitung in das Zugband 27 homogenisiert, und die Zug- und Biegebelastbarkeit der Schweißnaht deutlich erhöht. In alternativen Ausführungsformen sind spitzwinklige Schweißnähte oder mehrfach gezackte Schweißnähte denkbar, die ein vergleichbares Ergebnis liefern. Das Zugband 27 weist parallel zur Drehgelenksachse 1 eine wesentlich größere Breite als Dicke auf. Somit sind eine Elastizität und Biegbarkeit des Zugbands 27 bei der Rotation des zweiten Maulteils 10' gewährleistet. In Längsrichtung des Rohrschaftinstruments weist das Zugband 27 jedoch eine relativ hohe Steifigkeit auf, so dass auch Schubkräfte erzeugt werden können.

Durch das Ansetzen eines ersten Endes des Zugbands 27 an die konvexen Abschnitte 44, 44' der Gelenkführungsschienen 41, 41' wird sichergestellt, dass die Zugkraft, die mittels des Zugbands 27 ausgeübt wird, immer im wesentlichen tangential zur Kreisbewegung der gekrümmten Gelenkführungsschienen 41, 41' um die Drehgelenksachse 1 wirkt. Somit ist eine einheitliche, vom Öffnungswinkel unabhängige Kräfteübertragung sichergestellt. Ein zweites Ende des Zugbands 27 steht mit dem Handgriff 110 in Wirkverbindung und lässt sich mittels einer an diesem vorgesehenen Steuervorrichtung bewegen. Wegen der virtuellen Drehgelenksachse 1, die sich, wie bereits erläutert, außerhalb und oberhalb der Maulteile 10, 10' befindet, ist die Distanz zwischen der Drehgelenksachse 1 und dem ersten Ende des Zugbands 27 deutlich größer als die Distanz, die bei üblichen Gelenken erzielt wird. Daher besitzt die beschriebene Ausführungsform des Rohrschaftinstruments einen wesentlich größeren Hebel, mittels dessen das zweite Maulteil 10' über das Zugband 27 bewegt werden kann.

Zum Fixieren des Gewebes weisen die beiden Maulteile 10, 10' jeweils eine Klemmfläche 12, 12' auf. Das erste Maulteil 10 weist also an einem distalen Abschnitt eine nach oben gewandte erste Klemmfläche 12 auf. Diese erste Klemmfläche 12 ist quer zur Längsachse des ersten Maulteils 10 im Wesentlichen konkav ausgebildet. Im geschlossenen Zustand des Werkzeugkopfs 30 liegt die konvexe zweite Klemmfläche 12' des zweiten Maulteils 10' im Wesentlichen parallel zu dieser ersten Klemmfläche 12.

Im beschriebenen Ausführungsbeispiel sind diese Klemmflächen 12, 12' nicht nur dazu geeignet, das später zu schneidende Gewebe sicher zu fixieren, sondern bilden auch die Elektroden für einen Koagulationsvorgang. Hierfür sind Abschnitte der Klemmflächen 12, 12' elektrisch leitend und über Leiterbahnen mit einer HF-Stromquelle verbunden, die ebenfalls über den Handgriff 110 steuerbar ist. Somit kann vor dem Schneidvorgang das gefasst Gewebe so stark verödet werden, dass das Durchtrennen ohne eine Blutung möglich ist. Vorzugsweise werden zumindest Abschnitte der Maulteile 10, 10' im Spritzgussverfahren aus Keramik hergestellt. Somit lassen sich die Führungselemente, insbesondere die Gelenkführungsschienen 41, 41' und die Gelenkführungszapfen 42, 42' des Gelenks 40 einfach ausbilden. Das Gelenk 40 aus Keramik bildet eine elektrische Isolation zwischen den Maulteilen 10, 10', insbesondere zwischen deren Elektroden zur Koagulation.

Nach der Koagulation erfolgt im vorliegenden Ausführungsbeispiel der eigentliche mechanische Schneidvorgang. Hierfür wird parallel zu einer Fixierebene x-y (vgl. Fig. 11), die durch die Klemmflächen 12, 12' bestimmt wird, eine Schneidvorrichtung 50 bewegt. Diese Schneidvorrichtung 50 umfasst eine Schneide 51 zum Durchtrennen des Gewebes sowie einen Führungsdraht 52 mittels dessen eine Bewegung der Schneide 51 in Längsrichtung des Rohrschaftinstruments (x-Achse) möglich ist.

Vor dem Schneidvorgang ist die Schneide 51 so weit zum Rohrschaft 24 hin zurückgezogen, dass eine vorzeitige Verletzung des Gewebes nicht möglich ist. Vorzugsweise befindet sich die Schneide im ersten Maulteil 10 auf der Höhe der Gelenkführungszapfen 42, 42'. Aus dieser Ausgangsposition wird die Schneide 51 über eine in das zweite Maulteil 10' integrierte Rampe 55 (vgl. hierzu Fig. 4) auf die Fixierebene x-y zugeführt. Diese Rampe 55 befindet sich zwischen den beiden Gelenkführungsschienen 41, 41'. Für die Bewegung der Schneide 51 bzw. Klinge stellt das zweite Maulteil 10' eine Klingenführung 53 bereit. Diese Klingenführung 53 ist eine längliche Öffnung, die sich längs der Längsachse des zweiten Maulteils 10' erstreckt. Um die Schneide 51 senkrecht zur Fixierebene x-y zu halten, weist das zweite Maulteil 10' in seinem Mittelbereich Seitenteile 60, 60' auf, die derart parallel zu einander angeordnet sind, dass sie einen sich längs erstreckenden Kanal bilden. In diesem Kanal wird die Schneide 51 bzw. Klinge geführt.

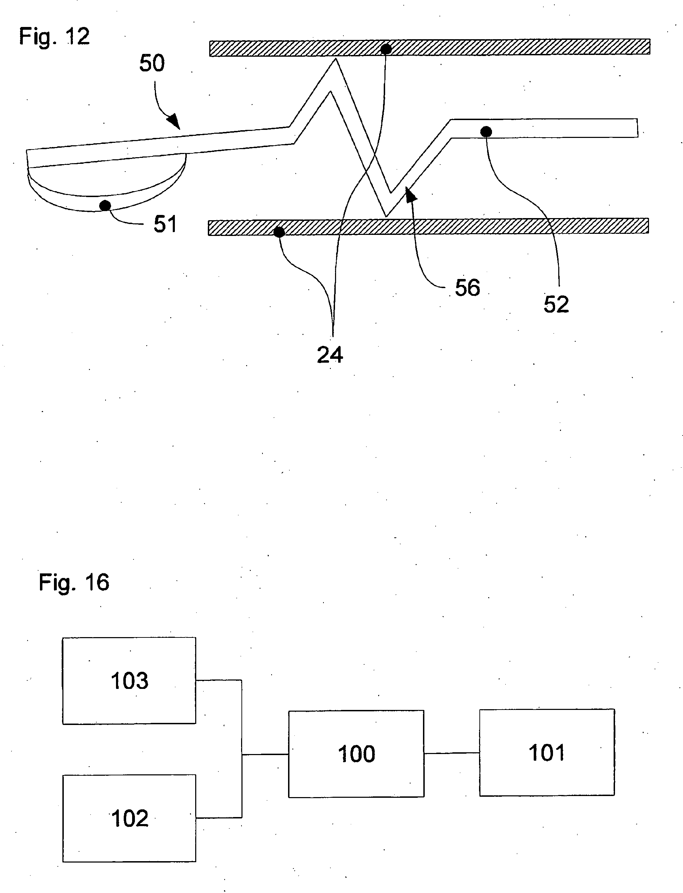

Nach dem Schließen der Maulteile 10, 10' gleitet die Schneide 51 also aus ihrer Ausgangsposition über die Rampe 55 in den besagten Kanal und kann dort in distale und proximale Richtung über das Gewebe gezogen bzw. geschoben werden. Um durch diese Bewegung eine schrittweise Durchtrennung des Gewebes zu gewährleisten, ist die Schneide 51 gegenüber der Fixierebene x-y vorgespannt. Eine Vorspanneinrichtung übt eine Kraft senkrecht zur Fixierebene x-y aus, die die Schneide 51 gegen die Ebene drückt. Diese Kraft wird über die Elastizität des Führungsdrahts 52 und dessen Biegung aufgebaut. Wie aus Fig. 12 ersichtlich, ist der Führungsdraht 52 in der von der Schneide 51 aufgespannten Ebene, senkrecht zur Fixierebene x-y, gekrümmt. In einem vorderen Abschnitt des Führungsdrahts 52 befindet sich eine Sicke 56. Die Sicke 56 ist derart in den Führungsdraht 52 integriert, dass sich bei vollständig ausgefahrenen Zustand der Schneidvorrichtung 50, also wenn sich die Schneide 51 am distalen Ende der Maulteile 10, 10' befindet, die Sicke im Rohrschaft 24 ebenfalls am distalen Ende dessen ist. Die Sicke 56 dient dazu, zumindest einen Teil der durch die Krümmung des Führungsdrahts 52 ausgeübten Kraft senkrecht zur Fixierebene x-y an den Rohrschaft 24 abzugeben und weist entsprechende Kontaktpunkte auf. Die Biegung des Führungsdrahts 52 ist so beschaffen, dass bei einem Parallelverlaufen des proximalen Endes des Führungsdrahts mit dem Rohrschaft 24 das distale Ende des freien Führungsdrahts 52 nach unten gebogen ist und die Schneide 51 zumindest teilweise unterhalb der Fixierebene x-y liegt. Der Führungsdraht 52 steht mit dem Handgriff 110 derart in Wirkverbindung, dass mittels diesem die Schneide 51 im Werkzeugkopf 30 hin- und herbewegt werden kann.

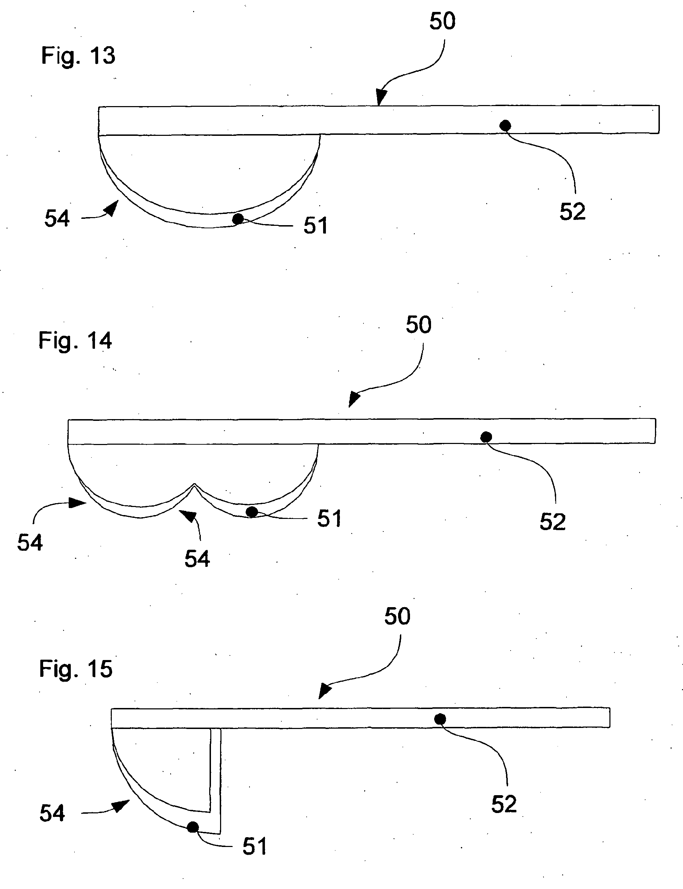

Hinsichtlich der Ausbildung der Schneide 51 sind verschiedenste Ausführungsformen denkbar. Diese werden im Weiteren anhand der Fig. 13, 14 und 15 beschrieben. Ein Gedanke der Erfindung besteht darin, dass die Schneide 51 mindestens einen Abschnitt aufweist, der im Wesentlichen parallel zur Fixierebene x-y und somit parallel zum fixierten Gewebe verläuft. Somit gleitet die Schneide 51 beim Schneidvorgang so lange über das Gewebe hinweg, bis dieses vollständig durchtrennt ist. Anders als bei herkömmlichen Schneidvorgängen kann so sichergestellt werden, dass selbst bei abgestumpfter Schneide 51 das Gewebe durchtrennt wird und nicht aufgrund des mechanischen Druckes zerquetscht wird. Der parallel zur Fixierebene x-y ausgebildete Abschnitt der Schneidklinge hat ebenfalls den Vorteil, dass die Schneide 51 nicht nur punktuell auf dem Gewebe ansetzt, sondern meist über einen längeren Bereich. Somit wird ein punktuelles Abnützen der Schneide 51 vermieden.

Fig. 13 zeigt eine halbkreisförmige Schneide 51, die eine konvexe Krümmung aufweist. Die Schneide 51 ist an der Unterseite des Führungsdrahts 52 angeordnet. Sie weist eine Schneidenkrümmung 54 in distaler und proximaler Richtung des Rohrschaftinstruments auf.

Fig. 14 zeigt eine Schneide 51, die aus zwei jeweils hintereinander angeordneten Halbkreisen besteht.

Fig. 15 zeigt eine Schneide 51, die in distaler Richtung eine Schneidenkrümmung 54 aufweist, und in proximaler Richtung einen zum Führungsdraht 52 senkrechten Abschnitt.

Vorzugsweise weist die Schneide 51 insgesamt eine Mikroverzahnung auf.

In einer alternativen Ausführungsförm (vgl. beispielsweise Fig. 10) handelt es sich bei dem Führungsdraht 52 um eine Schiene. Die Schiene kann derart ausgebildet sein, dass sie die gleiche Funktionalität wie der Führungsdraht 52 besitzt. Die Vorspannung gegenüber der Fixierebene x-y kann durch die Eigenelastizität der Schiene oder durch eine getrennte Vorrichtung (z.B. eine Feder) erreicht werden.

Die vorteilhafte Schneidvorrichtung 50 der Erfindung wurde bisher in Verbindung mit der vorteilhaften Gelenkausprägung beschrieben. Beide Erfindungen lassen sich jedoch auch getrennt von einander ausführen.

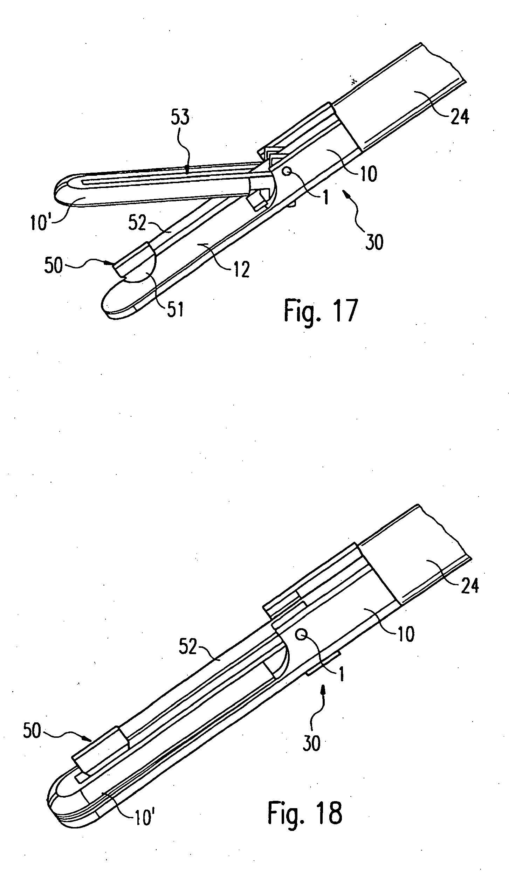

So zeigen beispielsweise die Fig. 17 und 18 die Schneidvorrichtung 50 in einem Werkzeugkopf 30, wobei das zweite Maulteil 10' nicht über eine Kulissenführung mit dem ersten Maulteil 10 in Wirkverbindung steht. Die Drehachse 1 liegt hier im Wesentlichen auf der Längsachse der Maulteile 10, 10'.

In einem erfindungsgemäßen Ausführungsbeispiel umfasst das Rohrschaftinstrument des Weiteren eine Schnittkontrollvorrichtung. Diese bestimmt, wann das Gewebe zwischen den beiden Klemmflächen 12, 12' vollständig durchtrennt ist. Bei dem Ausführungsbeispiel sitzt die Schneide 51 bei vollständig durchtrenntem Gewebe auf der ersten Klemmfläche 12 auf. Da die Klemmfläche 12 eine Elektrode zur Koagulation umfasst, ist sie zumindest abschnittsweise elektrisch leitend. Erfindungsgemäß ist zumindest ein Abschnitt der Schneide 51, der bei durchtrenntem Gewebe die Trennfläche 12 mechanisch kontaktiert, ebenfalls aus elektrisch leitendem Material ausgebildet. Mittels einer Schnittkontrollvorrichtung wird der elektrische Kontakt zwischen der Schneide 51 und der Klemmfläche 12 bestimmt. Das gefasste Gewebe gilt als vollständig durchtrennt, wenn bei einer kompletten Schnittbewegung von der Spitze 16' des zweiten Maulteils 10' bis zur Rampe 55 ein durchgehender elektrischer Kontakt zwischen Schneide 51 und Klemmfläche 12 besteht. Wie aus Fig. 16 ersichtlich, umfasst die Schnittkontrollvorrichtung zur Bestimmung sowie zur Anzeige des Fortschrittes des Schneidvorgangs eine Verarbeitungseinrichtung 100, eine Anzeigevorrichtung 101, einen Schalter 103 und einen Wegsensor 102. Der Wegsensor 102 bestimmt Position bzw. die Bewegung der Schneide 51 und hilft somit, ein Beobachtungsintervall zu definieren, das vorzugsweise eine komplette Schneidenbewegung umfasst. Der Schalter 103 wird im einfachsten Fall durch die elektrisch leitende Schneide 51 sowie die erste Klemmfläche 12 ausgebildet. Da das zu schneidende Gewebe eine gewisse elektrische Leitfähigkeit aufweist, gilt der elektrische Schalter 103 erst dann als geschlossen, wenn eine niederohmige Verbindung zwischen der Klemmfläche 12 und der Schneide 51 besteht. Eine entsprechende Vorrichtung ist der Verarbeitungsvorrichtung 100 vorgeschaltet. Stellt die Verarbeitungseinrichtung 100 fest, dass während eines kompletten Beobachtungsintervalls ein durchgehender niederohmiger Kontakt zwischen Schneide 51 und Klemmfläche 12 vorliegt, so zeigt sie dem Benutzer mittels der Anzeigevorrichtung 101 an, dass das gefasste Gewebe gänzlich durchtrennt ist. Somit wird die Schneidvorrichtung 50 geschont, da die Bewegung der Schneide 51 über die Klemmfläche 12 ohne dazwischen liegendes Gewebe diese schädigt.

Alternativ kann dem Benutzer auch stetig angezeigt werden, ob eine direkte mechanische Kontaktierung zwischen Schneide 51 und Klemmfläche 12 vorliegt. Da die Bewegung der Schneide 51 durch den Benutzer manuell durchgeführt wird, kann er selbstständig Rückschlüsse ziehen, ob das Gewebe ausreichend durchtrennt ist.

In einem weiteren Ausführungsbeispiel umfasst der Wegsensor 102 zwei elektrische Kontaktbereiche am distalen und proximalen Ende der Klingenführung 53, die derart ausgebildet sind, dass eine Kontaktierung zwischen der Schneide 51 und dem distalen Kontaktbereich sowie zwischen der Schneide 51 und dem proximalen Kontaktbereich bestimmt werden kann. Die Verarbeitungsvorrichtung 100 kann somit den Beginn und das Ende eines Beobachtungsintervalls erfassen.

Die Fig. 20 zeigt eine schematische Detailansicht des Handgriffs 110 aus Fig. 1. Der Handgriff 110 umfasst einen Handgriffkörper 117, an dessen Unterseite ein erster Griffhebel 122 integral ausgebildet ist. Dieser Griffhebel 122 weist eine Öffnung zur Aufnahme mehrerer Finger, vorzugsweise des Mittel-, Ring- und kleinen Fingers auf. Ein zweiter Griffhebel 122' ist nahe beim ersten Griffhebel 122 drehbeweglich mit dem Handgriffkörper 117 verbunden. Durch eine Bewegung des zweiten Griffhebels 122' relativ zum ersten Griffhebel 122 in proximale und distale Richtung, lassen sich die Maulteile 10, 10' des Werkzeugkopfs 30 öffnen und schließen. Die Griffhebel 122, 122' bilden einen Handabzug 120 und lassen sich so in der Hand des Benutzers aufnehmen, dass das gesamte Rohrschaftinstrument einhändig geführt werden kann. Hierfür umschließt die Hand Abschnitte der Griffhebel 122, 122'. An dem dem Handgriffkörper 117 abgewandten Ende des zweiten Griffhebels 122' befindet sich ein Fortsatz, der in eine Zahnstange 24 einrastet. Diese Zahnstange 124 ist in einem rechten Winkel zur Längsachse des ersten Griffhebels 122 an dessen dem Handgriffkörper 117 abgewandten Ende befestigt. Die Bezahnung der Zahnstange 124 ist derart ausgebildet, dass sich der zweite Griffhebel 122' schrittweise zum ersten Griffhebel 122 hinbewegen lässt und die entsprechende eingestellte Position ohne das permanente Ausüben einer Kraft hält. Um diese Fixierung der Griffhebel 122, 122' zueinander zu lösen, wird die Zahnstange 124 derart von dem Fortsatz 125 weggedrückt, dass diese nicht mehr in Eingriff stehen.

Des Weiteren weist der Handgriff 110 einen Fingerabzug 130 auf, der ebenfalls drehbeweglich an dem Handgriffkörper 117 befestigt ist. Durch die Betätigung des Fingerabzugs 130 kann die Schneidvorrichtung 50, insbesondere die Schneide 51, in distale Richtung bewegt werden. Ein nicht gezeigtes Federelement im Inneren des Handgriffkörpers 117 bringt den Fingerabzug 130 nach der Betätigung zurück in seine Ausgangsposition, wodurch die Schneidvorrichtung 50 in proximaler Richtung bewegt wird. Der Fingerabzug 130 ist distal vor dem ersten Griffhebel 122 derart angeordnet, dass bei einem Umgreifen der Griffhebel 122, 122' der Fingerabzug 130 mit dem Zeigefinger betätigbar ist.

Der Handgriff 110 weist einen Tastschalter 116 an der proximalen Seite des Handgriffkörpers 117 auf, der zur Steuerung des Koagulationsstroms dient. In einem alternativen Ausführungsbeispiel kann an Stelle des Tastschalters 116 eine Steuereinrichtung mit mehreren Betätigungselementen vorgesehen werden, mittels derer eine Vielzahl von Koagulationsmodi ausgewählt und durchgeführt werden können. Denkbar ist es ebenfalls, die Anzeigevorrichtung 101 am Handgriffkörper 117 vorzusehen.

In einer erfindungsgemäßen Ausführungsform sind Rohrschaft 24 und Handgriff 110 derart ausgebildet, dass der Rohrschaft 24 lösbar in den Handgriff 110 einlegbar ist. Hierfür befindet sich seitlich des Handgriffs 110 eine Aufnahmeöffnung 112, die mittels einer Abdeckung verschlossen werden kann.

Vor der Operation wird also ein steriler Einmal-Rohrschaft 24 mit entsprechendem Werkzeugkopf 30 und Schneidvorrichtung 50 in den wiederverwendbaren Handgriff 110 eingelegt und dort verriegelt. Ein Wiederverwenden des Rohrschafts 24 und der zugehörigen Vorrichtungen ist hier nicht vorgesehen. Zur mechanischen Anbindung des Werkzeugskopfs 30, der Schneidvorrichtung 51 und des Rohrschafts 24 weist der Handgriffkörper 117 ein erstes Kupplungselement 114 bzw. Kopplungselement, ein zweites Kupplungselement 114' bzw. Kopplungselement und ein drittes Kupplungselement 114" bzw. Kopplungselement auf. In das dritte Kupplungselement 114" greift ein an dem proximalen Ende des Rohrschafts 24 vorgesehener Ring derart ein, dass der Rohrschaft starr mit dem Handgriffkörper 117 verbunden ist. In das erste Kupplungselement 114, das mit dem zweiten Griffhebel 122' in Wirkverbindung steht, greift ein erster Innenrohradapter 22 mittels eines ebenfalls am proximalen Ende angeordneten Rings ein. Die Bewegung des zweiten Griffhebels 122' wird durch eine innerhalb des Handgriffkörpers 117 angeordnete Mechanik an das erste Kupplungselement 114 übertragen und überträgt diese wiederum an den ersten Innenrohradapter 22. Dieser steht direkt oder indirekt über das Zugband 27 mit dem zweiten Maulteil 10' in mechanischer Verbindung. Eine Längsverschiebung des ersten Innenrohradapters 22 gegenüber dem Rohrschaft 24 bewirkt also ein Öffnen und Schließen der Maulteile 10, 10'.

Ein zweiter Innenrohradapter 22' ist gegenüber dem ersten Innenrohradapter 22 frei beweglich in dessen Innerem angeordnet. Dieser Innenrohradapter 22' steht in Wirkverbindung mit dem Führungsdraht 52 und bewegt die Schneide 51. Durch das Einsetzen des Rohrschafts 24 in den Griffkörper 117 greift ein proximaler Ring am Ende des zweiten Innenrohradapters 22' in das zweite Kupplungselement 114' ein und überträgt die Bewegung bzw. die mittels des Fingerabzugs 130 ausgeübte Kraft an die Schneidvorrichtung 50.

Um das Einsetzen des Einmal-Rohrschafts 24 zu erleichtern, ist an diesem eine abnehmbare Fixierung vorgesehen, die die Innenrohradapter 22, 22' relativ zum Rohrschaft 24 in einer vorbestimmten Position hält, die derart ausgebildet ist, dass die Ringe einfach in die Kupplungselemente 114, 114', 114" einsetzbar sind.

Die Kupplungselemente 114, 114' 114" sind derart ausgebildet, dass der Rohrschaft 24 gegenüber dem Handgriff 110 rotiert werden kann. Somit kann die Ausrichtung des Werkezeugkopfs 30 relativ zum Handgriff 110 frei eingestellt werden. Bei der Rotation drehen sich die Ringe der Innenrohradapter 22, 22' und des Rohrschaftes 24 in den Kupplungselementen 114, 114', 114" und bilden so ein Drehgelenk aus.

Weiterhin sind folgende, nicht erfindungsgemäße, Ausführungsbeispiele denkbar:

### Ausführungsbeispiel 1:

Rohrschaftinstrument zum Greifen und/oder Koagulieren und/oder Durchtrennen von Gewebe,
wobei mindestens ein Gelenk 40 zum drehbeweglichen Lagern der Maulteile 10, 10' derart, dass die Maulteile 10, 10' aus einer Öffnungsstellung in eine Schließstellung bringbar sind, um das Gewebe mit den Klemmflächen 12, 12' zu fixieren,
wobei das Gelenk 40 derart ausgebildet ist, dass die Drehachse 1 des Gelenks 40 außerhalb der Maulteile 10, 10' liegt und das distale Ende mindestens eines Maulteils 10, 10' beim Öffnen von dem distalen Ende des Rohrschafts 24 wegbewegbar ist.

### Ausführungsbeispiel 2:

Rohrschaftinstrument nach einem der vorhergehenden Ausführungsbeispiele, wobei das Gelenk 40 eine Kulissenführung umfasst.

### Ausführungsbeispiel 3:

Rohrschaftinstrument nach einem der vorhergehenden Ausführungsbeispiele, wobei
das Gelenk 40 eine Gelenkführung an einem der beiden Maulteilen 10, 10' und mindestens eine Schiene oder Nut am anderen der beiden Maulteile 10, 10' umfasst.

### Ausführungsbeispiel 4:

Rohrschaftinstrument nach einem der vorhergehenden Ausführungsbeispiele,
wobei
mindestens zwei Teil-Gelenke 40, 40' zur Bildung eines, vorzugsweise mittig zwischen den Teil-Gelenken 40, 40' angeordneten, Durchlasses voneinander beabstandet vorgesehen sind.

### Ausführungsbeispiel 5:

Rohrschaftinstrument nach einem der vorhergehenden Ausführungsbeispiele,
wobei
eines der beiden Maulteile 10 starr mit dem Rohrschaft 24 verbunden ist.

### Ausführungsbeispiel 6:

Rohrschaftinstrument nach einem der vorhergehenden Ausführungsbeispiele,
wobei
zum Öffnen und Schließen eines zu bewegenden Maulteils 10' ein im Wesentlichen linear bewegbares Band vorgesehen ist, das mit einem elastischen Endabschnitt am zu bewegenden Maulteil 10'befestigt ist.

### Ausführungsbeispiel 7:

Rohrschaftinstrument nach einem der vorhergehenden Ausführungsbeispiele, insbesondere nach Ausführungsbeispiel 6,
wobei
das Band als Zugband 27, insbesondere aus Federstahl, ausgebildet ist, dessen Endabschnitt fest mit dem Maulteil 10' verbunden ist.

### Ausführungsbeispiel 8:

Rohrschaftinstrument nach einem der vorhergehenden Ausführungsbeispiele, insbesondere nach Ausführungsbeispiel 7,
wobei
das Band durch Schweißen am Maulteil 10' befestigt ist, wobei die Schweißnaht bevorzugt un-rechtwinklig und/oder bogenförmig zu einer Längsachse des Bandes verläuft.

### Ausführungsbeispiel 9:

Rohrschaftinstrument nach einem der vorhergehenden Ausführungsbeispiele,
wobei
mindestens eines der Maulteile 10, 10' zumindest im Bereich des Gelenks 40 aus elektrisch isolierendem Material, insbesondere aus Keramik, ist.

### Ausführungsbeispiel 10:

Rohrschaftinstrument nach einem der vorhergehenden Ausführungsbeispiele,
wobei
einen abnehmbaren Handgriff, umfassend
- ein erstes und ein zweites mechanisches Betätigungselement 120, 130,
- ein erstes Kraftübertragungselement 114, 114' zur abnehmbaren Kopplung des ersten Betätigungselements 120 an eine erste Funktionseinheit, insbesondere an mindestens eines der Maulteile 10, 10' des Werkzeugkopfs und
- ein zweites Kraftübertragungselement 114 zur abnehmbaren Kopplung des zweiten Betätigungselements 130 an eine zweite Funktionseinheit, insbesondere an eine Schneidvorrichtung 50 des Werkzeugkopfs 30.

### Ausführungsbeispiel 11:

Rohrschaftinstrument nach einem der vorhergehenden Ausführungsbeispiele, insbesondere nach Ausführungsbeispiel 10,
wobei
der Rohrschaft 24 und die Maulteile als Einmaleinheit ausgebildet sind und über die Kraftübertragungselemente 114, 114' austauschbar mit dem Handgriff 110 verbunden sind.

### Ausführungsbeispiel 12:

Rohrschaftinstrument nach einem der vorhergehenden Ausführungsbeispiele, insbesondere nach Ausführungsbeispiel 11,
wobei
die Einmaleinheit, insbesondere der Werkzeugkopf 30 und/oder die Maulteile 10, 10' und/oder die Schneidvorrichtung 50, vor dem Einbau in den Handgriff 110 mittels einer Fixierung derart vorpositioniert ist, dass die Kraftübertragungselemente 114, 114', 114" beim Einbau mit entsprechenden Gegenstücken in Eingriff bringbar sind.

### Bezugszeichenliste

- 1: Drehgelenksachse
- 2: Stoßkante
- 10, 10': Maulteil
- 12, 12': Klemmfläche
- 16, 16': Spitze
- 22, 22': Innenrohradapter
- 24: Rohrschaft
- 25: Adapter
- 27: Zugband
- 30: Werkzeugkopf
- 40: Gelenk
- 41, 41': Gelenkführungsschiene
- 42, 42': Gelenkführungszapfen
- 43, 43': konkaver Abschnitt der Gelenkführungsschiene
- 44, 44': konvexer Abschnitt der Gelenkführungsschiene
- 46, 46': Gelenkführungslager
- 50: Schneidvorrichtung
- 51: Schneide
- 52: Führungsdraht
- 53: Klingenführung
- 54: Schneidenkrümmung
- 55: Rampe
- 56: Sicke
- 60, 60': Seitenteil
- 100: Verarbeitungseinrichtung
- 101: Anzeigevorrichtung
- 102: Wegsensor
- 103: Schalter
- 110: Handgriff
- 112: Aufnahmeöffnung
- 114, 114', 114": Kupplungselement
- 116: Tastschalter
- 117: Handgriffkörper
- 120: Handabzüg
- 122, 122': Griffhebel
- 124: Zahnstange
- 125: Fortsatz
- 130: Fingerabzug
- x: x-Achse
- y: y-Achse
- z: z-Achse

## Patentansprüche

1. Rohrschaftinstrument, insbesondere elektrochirurgisches Rohrschaftinstrument,
zum Durchtrennen von Gewebe, umfassend:
- einen Rohrschaft (24),
- ein erstes und ein zweites Maulteil (10, 10') mit jeweils mindestens einer Klemmfläche (12, 12') zum Fixieren von Gewebe in einer Fixierebene (x-y), wobei mindestens eines der Maulteile (10, 10') mit dem Rohrschaft (24) verbunden ist,
- eine Schneidvorrichtung (50) mit einer Schneide (51), die zum Schneiden des fixierten Gewebes mittels einer Betätigungseinrichtung in einer Schnittrichtung (x) bewegbar ist, wobei die Schneide (51) mindestens einen Abschnitt umfasst, der parallel zur Fixierebene (x-y) verläuft,
wobei der besagte parallele Abschnitt der Schneide (51) im Wesentlichen parallel zur Fixierebene (x-y) beweglich geführt ist und
die Schneide (51) durch eine Vorspanneinrichtung (56) beim Schneiden gegen die Fixierebene (x-y) federelastisch vorgespannt ist,
**dadurch gekennzeichnet, dass**
die Vorspanneinrichtung (56) einen elastischen Führungsdraht (52) mit einer Biegung oder eine elastische Führungsschiene umfasst, wobei der Führungsdraht (52) oder die Führungsschiene mit der Schneide (51) im Wesentlichen starr verbunden ist und derart im Rohrschaft (24) geführt ist, dass die Schneide (51) gegenüber dem Rohrschaft (24) in Richtung auf die Fixierebene (x-y) hin vorgespannt ist.

2. Rohrschaftinstrument nach Anspruch 1,
**dadurch gekennzeichnet, dass**
die Vorspanneinrichtung (56) eine Sicke umfasst, die derart im Führungsdraht (52) angeordnet ist, dass sie bei vorgeschobener Schneide (51) nahe dem distalen Ende des Rohrschafts (24) ist.

3. Rohrschaftinstrument nach einem der vorhergehenden Ansprüche,
**dadurch gekennzeichnet, dass**
eines der beiden Maulteile (10, 10') eine sich entlang der Schnittrichtung (x) erstreckende Klingenführung (53) umfasst.

4. Rohrschaftinstrument nach einem der vorhergehenden Ansprüche,
**dadurch gekennzeichnet, dass**
die Schneide (51) mindestens abschnittsweise konvex gekrümmt ist.

5. Rohrschaftinstrument nach einem der vorhergehenden Ansprüche,
**gekennzeichnet durch**
eine insbesondere rampenförmige Klingenführung (53), die derart ausgebildet und angeordnet ist, dass sie die Schneide (51) aus einer zur Fixierebene (x-y) beabstandeten Ausgangsposition **durch** eine Bewegung in der Schnittrichtung (x) auf die Fixierebene (x-y) zuführt.

6. Rohrschaftinstrument nach Anspruch 3 oder Anspruch 5,
**dadurch gekennzeichnet, dass**
die Maulteile (10, 10') drehbeweglich zueinander angeordnet sind und
die Klingenführung (53) derart ausgebildet ist, dass die Schneide (51) in eine Ausgangsposition nahe der Drehachse der Maulteile (10, 10') bringbar ist.

7. Rohrschaftinstrument nach einem der vorhergehenden Ansprüche,
**dadurch gekennzeichnet, dass**
die Klemmflächen (12, 12') Elektroden zum Zuführen von HF-Koagulationsströmen zu dem gefassten Gewebe umfassen.

8. Rohrschaftinstrument nach einem der vorhergehenden Ansprüche,
**dadurch gekennzeichnet, dass**
die Schneide (51) mindestens abschnittsweise eine Mikroverzahnung umfasst.

## Claims

1. Tubular shaft instrument, in particular an electrosurgical tubular shaft instrument, for separating tissue, comprising:
- a tubular shaft (24),
- a first and a second mouth part (10, 10'), each with at least one clamping surface (12, 12') for fixing tissue in a fixing plane (x-y), wherein at least one of the mouth parts (10, 10') is connected to the tubular shaft (24),
- a cutting device (50) with a blade (51) which, in order to cut the fixed tissue, is movable in a cutting direction (x) by means of an actuating device, wherein the blade (51) comprises at least one portion that extends parallel to the fixing plane (x-y),
wherein said parallel portion of the blade (51) is movably guided substantially parallel to the fixing plane (x-y), and
the blade (51) is resiliently prestressed against the fixing plane (x-y) by a prestressing device (56) during cutting,
**characterized in that** the prestressing device (56) comprises an elastic guide wire (52) with a bend, or an elastic guide rail, wherein the guide wire (52) or the guide rail is substantially rigidly connected to the blade (51) and is guided in the tubular shaft (24) in such a way that the blade (51) is prestressed with respect to the tubular shaft (24) in the direction of the fixing plane (x-y).

2. Tubular shaft instrument according to Claim 1, **characterized in that** the prestressing device (56) comprises a bead, which is arranged in the guide wire (52) in such a way that said bead is near the distal end of the tubular shaft (24) when the blade (51) is pushed forwards.

3. Tubular shaft instrument according to one of the preceding claims, **characterized in that** one of the two mouth parts (10, 10') comprises a blade guide (53) extending along the cutting direction (x).

4. Tubular shaft instrument according to one of the preceding claims, **characterized in that** the blade (51) has a convex curvature at least in part.

5. Tubular shaft instrument according to one of the preceding claims, **characterized by** an in particular ramp-shaped blade guide (53), which is designed and arranged in such a way that it brings the blade (51) from a starting position, at a distance from the fixing plane (x-y), onto the fixing plane (x-y) by means of a movement in the cutting direction (x).

6. Tubular shaft instrument according to Claim 3 or Claim 5, **characterized in that** the mouth parts (10, 10') are arranged rotationally movable to each other, and the blade guide (53) is designed in such a way that the blade (51) can be brought to a starting position near the axis of rotation of the mouth parts (10, 10').

7. Tubular shaft instrument according to one of the preceding claims, **characterized in that** the clamping surfaces (12, 12') comprise electrodes for delivering HF coagulation currents to the gripped tissue.

8. Tubular shaft instrument according to one of the preceding claims, **characterized in that** the blade (51) comprises a micro-toothing at least in part.

## Revendications

1. Instrument à tige tubulaire, en particulier instrument à tige tubulaire électrochirurgical, pour découper des tissus, comprenant :
- une tige tubulaire (24),
- une première et une deuxième partie de mâchoire (10, 10') avec à chaque fois au moins une surface de serrage (12, 12') pour fixer des tissus dans un plan de fixation (x-y), au moins l'une des parties de mâchoire (10, 10') étant connectée à la tige tubulaire (24),
- un dispositif de coupe (50) avec un tranchant (51) qui peut être déplacé dans une direction de coupe (x) au moyen d'un dispositif d'actionnement pour couper les tissus fixés, le tranchant (51) comprenant au moins une portion qui s'étend parallèlement au plan de fixation (x-y),
ladite portion parallèle du tranchant (51) étant guidée de manière déplaçable essentiellement parallèlement au plan de fixation (x-y) et
le tranchant (51) étant précontraint de manière élastique à ressort contre le plan de fixation (x-y) par un dispositif de précontrainte (56) lors de la coupe,
**caractérisé en ce que**
le dispositif de précontrainte (56) comprend un fil de guidage élastique (52) avec une partie courbe ou un rail de guidage élastique, le fil de guidage (52) ou le rail de guidage étant connecté de manière essentiellement rigide au tranchant (51) et étant guidé dans la tige tubulaire (24) de telle sorte que le tranchant (51) soit précontraint dans la direction du plan de fixation (x-y) par rapport à la tige tubulaire (24).

2. Instrument à tige tubulaire selon la revendication 1,
**caractérisé en ce que**
le dispositif de précontrainte (56) comprend une moulure qui est disposée dans le fil de guidage (52) de telle sorte que lorsque le tranchant (51) est avancé, elle se situe à proximité de l'extrémité distale de la tige tubulaire (24).

3. Instrument à tige tubulaire selon l'une quelconque des revendications précédentes,
**caractérisé en ce que**
l'une des deux parties de mâchoire (10, 10') comprend un guide de lame (53) s'étendant le long de la direction de coupe (x).

4. Instrument à tige tubulaire selon l'une quelconque des revendications précédentes,
**caractérisé en ce que**
le tranchant (51) a au moins en partie une courbure convexe.

5. Instrument à tige tubulaire selon l'une quelconque des revendications précédentes,
**caractérisé par**
un guide de lame (53) en particulier en forme de rampe, qui est réalisé et disposé de telle sorte qu'il amène le tranchant (51) depuis une position de départ espacée du plan de fixation (x-y) par un mouvement dans la direction de coupe (x) jusqu'au plan de fixation (x-y).

6. Instrument à tige tubulaire selon la revendication 3 ou la revendication 5,
**caractérisé en ce que**
les parties de mâchoire (10, 10') sont disposées de manière mobile en rotation l'une par rapport à l'autre et le guide de lame (53) est réalisé de telle sorte que le tranchant (51) puisse être amené dans une position de départ à proximité de l'axe de rotation des parties de mâchoire (10, 10').

7. Instrument à tige tubulaire selon l'une quelconque des revendications précédentes,
**caractérisé en ce que**
les surfaces de serrage (12, 12') comprennent des électrodes pour acheminer des flux de coagulation HF aux tissus saisis.

8. Instrument à tige tubulaire selon l'une quelconque des revendications précédentes,
**caractérisé en ce que**
le tranchant (51) comprend au moins en partie une microdenture.
